# EUROPÄISCHE PATENTANMELDUNG

(11) **EP 3 574 823 A1**
(43) Veröffentlichungstag der Anmeldung: **04.12.2019**
(21) Anmeldenummer: 18174901.1
(22) Anmeldetag: 29.05.2018
(51) Int. Cl.: A61B 5/00, A61B 5/113, A61B 6/00, G01R 33/567, A61B 5/055, A61B 6/03

(54) **VERFAHREN ZUR MESSUNG EINER ATMUNGSBEWEGUNG, VERFAHREN ZUR AUFNAHME VON MEDIZINISCHEN BILDDATEN, MESSEINRICHTUNG UND MEDIZINISCHE BILDGEBUNGSEINRICHTUNG**

(71) Anmelder: Siemens Healthcare GmbH, 91052 Erlangen (DE)
(72) Erfinder: Batzer, Ulrich, 91054 Buckenhof (DE)

(57) **Zusammenfassung**

Verfahren zur Messung eines wenigstens eine Atmungsbewegung eines auf einer Patientenlagerungseinrichtung (15) gelagerten Patienten (17) beschreibenden Messsignals, dadurch gekennzeichnet, dass auf der Patientenlagerungseinrichtung (15) zumindest bereichsweise unterhalb des Patienten (17) ein komprimierbares Druckkissen (2) einer Druckkisseneinrichtung angeordnet wird, wobei in einem Inneren (6) des Druckkissens (2) ein Innendruck vorhanden ist oder erzeugt wird und das Messsignal aus einer Druckverlaufsmessung des Innendrucks eines mit dem Inneren (6) des Druckkissens (2) kommunizierenden Drucksensors (5) der Druckkisseneinrichtung bestimmt wird.

## Beschreibung

Die Erfindung betrifft ein Verfahren zur Messung eines wenigstens eine Atmungsbewegung eines auf einer Patientenlagerungseinrichtung gelagerten Patienten beschreibenden Messsignals. Weiterhin betrifft die Erfindung ein Verfahren zur Aufnahme von medizinischen Bilddaten eines Patienten sowie eine Messeinrichtung und eine medizinische Bildgebungseinrichtung.

Für eine präzise diagnostische Bildgebung von Organen im Oberkörper eines Patienten ist es notwendig, dass diese während der Bildgebung möglichst bewegungsfrei erfasst werden können. Dazu ist es bekannt, dass der Patient in Ruhelage auf einer Patientenlagerungseinrichtung gelagert wird und je nach der für die Bildgebung verwendeten Bildgebungsmodalität zu bestimmten Atemmanövern aufgefordert wird. In manchen Bildgebungsmodalitäten ist jedoch aufgrund der langen Dauer der Bildaufnahme ein dauerhaftes Atemmanöver, wie beispielsweise das Anhalten von Luft, nicht möglich. Auch gibt es Patienten, welche nicht in der Verfassung sind, derartige Atemmanöver durchzuführen. Um atmungsinduzierte Bewegungsstörungen bei der Bildgebung kompensieren zu können, wird die Atmung des Patienten gemessen.

Es ist bekannt, dass eine derartige Messung mit einem Brustgurt durchgeführt werden kann, welcher um den Oberkörper des Patienten gelegt wird. Eine derartige Atmungsmessung mit einem Brustgurt ist umständlich zu handhaben, da die Brustgurte direkt an dem Patienten angeordnet werden müssen. Dazu müssen verschiedene Brustgurte vorgehalten werden, welche abhängig vom Patientenumfang eingesetzt werden können. Je nach Verfassung des Patienten kann eine Applikation eines solchen Brustgurtes nur schwer zu handhaben sein und einen nahen Kontakt zwischen Personal und Patient erfordern, welcher in manchen Situationen als kritisch anzusehen ist. Weiterhin müssen die Brustgurte nach jedem Einsatz gereinigt werden, was sich aufgrund des textilen Materials des Brustgurtes aufwändig gestaltet. Außerdem müssen die verschiedenen Brustgurte regelmäßig gewechselt werden, da sie ihre für die Atmungsmessung notwendige Spannkraft verlieren. Andere Methoden zur Atmungserfassung umfassen optische oder radarbasierte Verfahren, welche die Atmungsbewegung jedoch nicht so unmittelbar wie ein Brustgurt messen und daher anfälliger für Fehlmessungen sind. Auch benötigen derartige Methoden eine aufwändige Applikation, um gute Ergebnisse liefern zu können. Darüber hinaus sind die Kosten für Anschaffung und Betrieb bei derartigen Verfahren vergleichsweise hoch.

Für eine Atmungsmessung ist es wünschenswert, dass die dazu eingesetzten Messmittel eine Bildgebung nicht behindern oder beeinträchtigen. Dies erfordert beispielsweise in einem Magnetresonanztomographen die Verwendung von nicht oder nur vernachlässigbar gering magnetischen Messmitteln bzw. bei auf Röntgenstrahlen basierenden Bildgebungsmodalitäten, wie einem Computertomographen, die Verwendung von möglichst röntgentransparenten Materialien.

Der Erfindung liegt die Aufgabe zugrunde, ein Verfahren zur Messung eines wenigstens eine Atmungsbewegung eines auf einer Patientenlagerungseinrichtung gelagerten Patienten beschreibenden Messsignals anzugeben, welches einfach durchzuführen ist und keinen nennenswerten Einfluss auf gängige Bildgebungsverfahren hat.

Zur Lösung dieser Aufgabe ist erfindungsgemäß für ein Verfahren der eingangs genannten Art vorgesehen, dass auf der Patientenlagerungseinrichtung zumindest bereichsweise unterhalb des Patienten ein komprimierbares Druckkissen einer Druckkisseneinrichtung angeordnet wird, wobei in einem Inneren des Druckkissens ein Innendruck vorhanden ist oder erzeugt wird und das Messsignal aus einer Druckverlaufsmessung eines zeitlichen Verlaufs des Innendrucks eines mit dem Inneren des Druckkissens kommunizierenden Drucksensors der Druckkisseneinrichtung bestimmt wird.

Das Einatmen und das Ausatmen des Patienten erzeugen jeweils eine Bewegung des Patientenkörpers, wobei sich beispielsweise beim Einatmen durch eine Bewegung der Rippen und des Zwerchfells der Brustraum zur Aufnahme der eingeatmeten Luft in den Lungen vergrößert. Beim Ausatmen erfolgt entsprechend eine umgekehrte Bewegung und eine Volumenabnahme des Oberkörpers.

Diese Atembewegung des Patienten kann durch ein Druckkissen gemessen werden, welches unterhalb des Patienten, also zwischen der Patientenlagerungseinrichtung, auf der sich der Patient befindet, und dem Patienten selbst angeordnet wird. Das Druckkissen ist komprimierbar und weist dabei einen Innendruck auf, welcher gleich oder größer als der Umgebungsdruck ist. Die Erzeugung des Innendrucks kann mechanisch, beispielsweise durch eine expandierende Struktur, oder pneumatisch, beispielsweise durch ein Befüllen des Druckkissens mit einem insbesondere gasförmigen Medium erfolgen. Die Atembewegung des auf dem Druckkissen positionierten Patienten erzeugt eine Kompression des Druckkissens, welche als Veränderung des Innendrucks über den Drucksensor der Druckkisseneinrichtung messbar ist. Der Innendruck des Druckkissens ändert sich folglich aufgrund der Atembewegung des Patienten. Eine oder mehrere Atmungsbewegungen, welche jeweils einem Einatmen bzw. einem Ausatmen entsprechen, können durch eine Druckverlaufsmessung des Innendrucks des Druckkissens bestimmt werden. Aus einer Druckverlaufsmessung, durch welche innerhalb eines Messzeitraumes mehrere Atmungsbewegungen des Patienten erfasst werden, kann auch eine Atemfrequenz des Patienten bestimmt werden.

Der zeitliche Verlauf des Innendrucks kann durch den mit dem Inneren des Druckkissens kommunizierenden Drucksensor der Druckkisseneinrichtung bestimmt werden. Der Drucksensor kann dabei ein Teil des Druckkissens sein und beispielsweise im Inneren des Druckkissen angeordnet sein oder er kann über ein Verbindungselement, beispielsweise einen Schlauch, mit dem Druckkissen verbunden sein.

Aufgrund der Platzierung des Druckkissens zwischen dem Patienten und der Patientenlagerungseinrichtung ergibt sich eine definierte Patientenumgebung mit einer quasi-statischen Pose des Patienten. Das Körpergewicht des Patienten wirkt dabei vorteilhaft als ein Gegengewicht für eine einseitige Druckbelastung des Patientenkörpers durch das Druckkissen bzw. während einer Messung des Innendrucks des Druckkissens durch den Drucksensor. Die aufgrund der Atmung des Patienten erzeugte Atmungsbewegung wird auf das Druckkissen übertragen und verursacht eine Änderung des Innendrucks des Druckkissens.

Das erfindungsgemäße Verfahren bietet den Vorteil, dass für die Messung nur ein geringer Materialaufwand erforderlich ist und sich das Druckkissen vorteilhaft ohne metallische oder magnetische Bauteile realisieren lässt, so dass insbesondere in dem für die Bildgebung relevanten Bereich keine oder keine nennenswerte Beeinflussung der Bildgebung erfolgt. Weiterhin hat es sich gezeigt, dass es für die Erfassung der Atmungsbewegung nicht erforderlich ist, dass der Patient entkleidet wird, da auch durch ein oder mehrere Schichten von Kleidungen noch eine Änderung des Innendrucks infolge der Atmungsbewegung des Patienten erfassbar ist. Darüber hinaus ist auch die Anordnung des Druckkissens zwischen dem Patienten und der Patientenlagerungseinrichtung einfacher umzusetzen und für den Patienten weniger einschränkend als das Anlegen eines Brustgurtes für eine Atmungsmessung. Auch ist die Messung der Atmungsbewegung mittels des Druckkissens weitgehend unabhängig von einem Umfang des Patienten, so dass auf das Vorhalten von Messmitteln in verschiedenen Größen vorteilhaft verzichtet werden kann. Das erfindungsgemäße Verfahren bietet somit den Vorteil, dass es insbesondere während einer medizinischen Bildgebung sehr einfach anzuwenden ist und nur eine vergleichsweise geringe Einschränkung für den Patienten bedeutet.

Erfindungsgemäß kann vorgesehen sein, dass das Druckkissen zumindest bereichsweise unterhalb eines Rückens des Patienten, insbesondere gegenüber einer Brust oder eines Bauchs des Patienten, angeordnet wird. Die Platzierung kann dabei rückenseitig mehr gegenüber einer Brust des Patienten bzw. mehr gegenüber eines Bauchs des Patienten erfolgen, je nachdem, in welcher Region der Patient die stärkste Atmungsbewegung zeigt. Die Positionierung des Druckkissens unterhalb eines Rückens des Patienten ist sehr einfach umzusetzen und erzeugt keine nennenswerte Beeinträchtigung des Wohlbefindens des Patienten auch bei länger andauernden Messungen.

Für die Erzeugung des Innendrucks kann erfindungsgemäß vorgesehen sein, dass der Innendruck durch einen Schaumstoffkörper des Druckkissens erzeugt wird. Der Schaumstoffkörper ist dabei komprimierbar und beispielsweise teilweise komprimiert in einer Hülle des Druckkissens positioniert, so dass er sowohl einen Innendruck des Druckkissens erzeugt als auch eine weitere Kompression des Schaumstoffkörpers durch die Atmungsbewegung des auf dem Druckkissen gelagerten Patienten ermöglicht wird.

Erfindungsgemäß kann bei einem Druckkissen mit Schaumstoffkörper vorgesehen sein, dass als Drucksensor wenigstens ein druckempfindlicher Widerstand verwendet wird. Durch den wenigstens einen druckempfindlichen Widerstand kann eine Kompression des Druckkissens, insbesondere des Schaumstoffkörpers des Druckkissens, infolge einer Atmungsbewegung des Patienten gemessen werden.

In einer bevorzugten Ausgestaltung der Erfindung kann es vorgesehen sein, dass ein aufblasbares Druckkissen verwendet wird, wobei der Innendruck durch eine Pumpe der Druckkisseneinrichtung erzeugt wird. Durch die Pumpe kann ein Medium, beispielsweise Luft, in das Innere des Druckkissens gepumpt werden, um einen definierten Innendruck im Inneren des Kissens zu erzeugen. Die Pumpe kann dabei beispielsweise über wenigstens einen Schlauch an das Druckkissen angeschlossen sein und dadurch mit dem Inneren des Druckkissens kommunizieren. Ebenso kann ein Drucksensor zur Messung des Innendrucks des Kissens bzw. des Drucks des Mediums im Inneren des Druckkissens über einen Schlauch mit dem Kissen verbunden sein und so mit dem Inneren des Druckkissens kommunizieren. Die Möglichkeit, die Pumpe oder den Sensor über Schläuche mit dem Druckkissen zu verbinden, bietet die Möglichkeit, die Pumpe und/oder den Drucksensor beabstandet von dem Kissen anzuordnen, so dass insbesondere in dem für die Bildgebung relevanten Bereich keine oder keine nennenswerte Beeinflussung der Bildgebung erfolgt. Die für die Messung relevante Information der Innendruckveränderung des Druckkissens lässt sich mit Hilfe eines Schlauchs sehr gut über eine gewisse Distanz leiten, so dass der Drucksensor der Druckkisseneinrichtung ohne Einbußen in der Messqualität beabstandet zu dem Druckkissen und somit auch gegebenenfalls beabstandet zu einer Bildgebungseinrichtung platziert werden kann.

Dabei kann für den Innendruck weiterhin erfindungsgemäß vorgesehen sein, dass eine Höhe des Innendrucks in Abhängigkeit einer wenigstens eine Eigenschaft des Patienten beschreibenden Patienteninformation bestimmt wird. Die Patienteninformation kann dabei für den auf der Patientenlagerungseinrichtung positionierten Patienten, an dem die Messung der Atmungsbewegung vorgenommen wird, beispielsweise eine Größe des Patienten, ein Gewicht des Patienten und/oder ein Alter des Patienten beschreiben. Dies bietet den Vorteil, dass für eine optimale Messung der Pulswelle der Innendruck des Druckkissens den jeweilig zu untersuchenden Patienten angepasst werden kann. Eine Auswertung der Patienteninformation und/oder eine Ansteuerung der Pumpe zur Erzeugung eines Innendrucks in der abhängig von der Patienteninformation bestimmten Höhe kann durch eine Recheneinrichtung vorgenommen werden, wobei die Recheneinrichtung beispielsweise eine Recheneinrichtung der Druckkisseneinrichtung sein kann. Es ist möglich, dass durch die Pumpe sowohl Luft in das Innere des Druckkissens gepumpt werden kann, um den Innendruck des Druckkissens zu erhöhen, als auch dass über die Pumpe Luft aus dem Inneren des Druckkissens abgelassen werden kann, um einen im Inneren des Druckkissens herrschenden Innendruck zu reduzieren, so dass eine Steuerung bzw. eine Regelung unter Zuhilfenahme des den Innendrucks messenden Drucksensors möglich ist.

Erfindungsgemäß kann vorgesehen sein, dass ein Innendruck zwischen 100 mbar und 400 mbar verwendet wird. Der Innendruck ist dabei als Überdruck gegenüber einem Umgebungsdruck zu verstehen, so dass beispielsweise bei einem mit Luft gefüllten Druckkissen ein Innendruck vorhanden ist oder erzeugt wird, welcher zwischen 100 mbar und 400 mbar höher als ein Umgebungsluftdruck ist.

In einer bevorzugten Ausgestaltung der Erfindung kann vorgesehen sein, dass ein aufblasbares Druckkissen verwendet wird, dessen Kontaktfläche zu der Patientenlagerungseinrichtung und/oder dessen zum Patienten weisende Oberfläche jeweils in zumindest teilweise aufgeblasenem Zustand zwischen 50 cm² und 200 cm² beträgt und/oder dessen Dicke in zumindest teilweise aufgeblasenem Zustand zwischen 0,1 cm und 10 cm beträgt. Das Druckkissen kann z. B. eine näherungsweise zylinderförmige bzw. zylinderscheibenförmige Form aufweisen. Das Druckkissen umfasst zwei Oberflächen, welche durch ein Aufblasen des Druckkissens um eine Dicke des Druckkissens voneinander beabstandet werden können. Bei einem zwischen dem Patienten und der Patientenlagerungseinrichtung angeordneten Druckkissen weist eine Oberfläche des Kissens zum Patienten und die gegenüberliegende Oberfläche ist in Kontakt mit der Patientenlagerungseinrichtung. Das Druckkissen kann eine Dicke von wenigen Millimetern in einem entleerten Zustand aufweisen sowie bei einer Befüllung mit dem maximalen Innendruck eine Dicke von bis zu 10 cm aufweisen, um ein mögliches Hohlkreuz oder ähnliche Fehlstellungen des Patienten kompensieren zu können. Die zum Patienten weisende Oberfläche sowie die zur Patientenlagerungseinrichtung weisende Oberfläche des Druckkissens können jeweils einen Flächeninhalt zwischen 50 cm² und 200 cm² aufweisen. Es ist möglich, dass die Druckkisseneinrichtung mehrere Druckkissen umfasst, welche an den Drucksensor und/oder eine gegebenenfalls vorhandene Pumpe angeschlossen werden können, so dass abhängig von dem Patienten ein Kissen mit einer passenden Oberfläche bzw. einer passenden Dicke gewählt werden kann.

Erfindungsgemäß kann vorgesehen sein, dass ein Druckkissen aus einem röntgentransparenten Material und/oder einem amagnetischen Material, insbesondere aus Kunststoff, verwendet wird. Ein amagnetisches Material bezeichnet in diesem Zusammenhang ein Material, welches nicht oder im Wesentlichen nicht magnetisch ist und durch dessen Platzierung zwischen Patienten und Patientenlagerungseinrichtung kein nennenswerter Einfluss auf eine Magnetresonanztomographiemessung erfolgt, bzw. welches keine nennenswerte Erwärmung während einer Magnetresonanztomographiemessung erfährt. Als röntgentransparentes Material ist in diesem Zusammenhang ein röntgentransparentes oder im Wesentlichen röntgentransparentes Material zu verstehen, welches einen Einsatz des Druckkissens während einer Röntgenbildgebung ermöglicht, ohne dass durch das zwischen dem Patienten und der Patientenlagerungseinrichtung positionierte Druckkissen ein nennenswerter Einfluss auf die durch die Röntgenbildgebung erzeugten Bilddaten erfolgt. Insbesondere kann dabei ein Druckkissen aus Kunststoff verwendet werden, da vielfältige Kunststoffe bekannt sind, welche weder Magnetresonanztomographiemessungen noch Röntgenbildgebung nennenswert beeinflussen.

In einer bevorzugten Ausgestaltung der Erfindung kann vorgesehen sein, dass ein Druckkissen verwendet wird, welches in die Patientenlagerungseinrichtung integriert ist. Das Druckkissen kann dabei mit einer Oberfläche an der Patientenlagerungseinrichtung befestigt werden und/oder zumindest teilweise in einer Aufnahme der zur Patientenlagerung vorgesehenen Patientenlagerungsfläche der Patientenlagerungseinrichtung aufgenommen sein. Es ist insbesondere möglich, dass die Patientenlagerungseinrichtung mehr als ein integriertes Druckkissen umfasst, wobei eines dieser Druckkissen ausgewählt und für die Messung der Pulswelle verwendet wird. Bei der Auswahl des Druckkissens kann dabei berücksichtigt werden, ob eine Atmungsbewegung des Patienten beispielsweise stärker in einem Bereich unterhalb des Bauchs des Patienten bzw. in einem Bereich unterhalb der Brust des Patienten auftritt, so dass ein Kissen verwendet werden kann, mit welchem eine möglichst große Atmungsbewegung des Patienten aufgenommen und als Veränderung des Innendrucks messbar gemacht werden kann.

Erfindungsgemäß kann vorgesehen sein, dass die Druckverlaufsmessung um den Einfluss eines einen Herzschlag des Patienten beschreibenden Pulssignals, insbesondere eines Elektrokardiogramm-Signals und/oder eines pneumatisch gemessenen Pulssignals, kompensiert wird. Auch aufgrund des Herzschlags des Patienten, welcher durch den systolischen Blutausstoß eine Pulswelle erzeugt, die sich als Druckänderung bzw. Volumenänderung des Körpers des Patienten, insbesondere der Blutgefäße des Patienten, ausbildet, kann eine Kompression des Druckkissens hervorgerufen werden. Um die durch den Herzschlag erzeugte Körperbewegung, beispielsweise im Fall einer nur geringen Atmungsbewegung des Patienten, kompensieren zu können, kann vorgesehen sein, dass ein den Herzschlag des Patienten beschreibendes Pulssignal herangezogen wird. Dieses Pulssignal kann beispielsweise in bekannter Weise mittels einer Elektrokardiogrammmessung erzeugt werden. Es ist auch möglich, dass das Druckkissen oder ein weiteres Druckkissen verwendet wird, um aus der Veränderung des Innendrucks, welcher auf die durch den Herzschlag des Patienten erzeugte Pulswelle zurückzuführen ist, ein Herzschlag des Patienten bestimmt wird. Die Kompensation des Einflusses des Herzschlags des Patienten auf die Druckverlaufsmessung zur Bestimmung der Atmungsbewegung kann beispielsweise durch Echo-Cancellation erfolgen. Auf diese Weise kann die Signalqualität des aus der Druckverlaufsmessung erzeugten, die Atmungsbewegung des Patienten beschreibenden Messsignals verbessert werden.

Vorteilhaft kann für die Messung des Pulssignals eine pneumatische Messung einer Pulswelle des Patienten eingesetzt werden. Eine derartige Messung kann beispielsweise mittels eines weiteren Druckkissens erfolgen, welches derart unter dem Patienten positioniert wird, dass eine durch die Pulswelle hervorgerufene Volumenänderung, insbesondere eines Blutgefäßes, auf das weitere Druckkissen übertragen wird. Es ist denkbar, dass dazu eine Patientenlagerungseinrichtung verwendet wird, welche wenigstens ein Druckkissen sowie wenigstens ein weiteres Druckkissen umfasst. Die Druckkissen können dabei derart an der Patientenlagerungseinrichtung angeordnet sein, dass der Patient so auf der Patientenlagerungseinrichtung angeordnet werden kann, dass das wenigstens eine Druckkissen zur Messung der Atmungsbewegung des Patienten unterhalb des Rückens und gegenüberliegend von einer Brust bzw. eines Bauchs des Patienten angeordnet ist und dass das wenigstens eine weitere Druckkissen zur Messung der Pulswelle beispielsweise unterhalb eines Oberschenkels, insbesondere unterhalb einer Oberschenkelarterie des Patienten, positioniert ist. Durch eine derartige Patientenlagerungseinrichtung können sowohl die Messung der Atmungsbewegung als auch die Messung des Pulssignals in einfacher Weise und komfortabel für den Patienten umgesetzt werden.

Für die Bestimmung des Messsignals kann erfindungsgemäß vorgesehen sein, dass das Messsignal durch eine Grenzwerterkennung des von dem Drucksensor gemessenen Innendrucks des Druckkissens erfolgt. Das Messsignal kann dabei angeben, wann eine Atmungsbewegung des Patienten vorliegt und wann nicht. Durch eine Verlaufsmessung des Innendrucks des Druckkissens kann somit eine Erkennung eines Atemvorgangs des Patienten erfolgen. Die Bestimmung einzelner Atmungsvorgänge des Patienten kann beispielsweise zur Triggerung bzw. zur Auslösung von Bildaufnahmen einer medizinischen Bildgebungseinrichtung erfolgen. Die Grenzwerterkennung kann beispielsweise durch Festlegen eines Grenzwertes des Innendrucks durchgeführt werden, wobei der Grenzwert einen Wert des Innendrucks angibt, ab dem bei einer Mehrheit der Patienten eine Atmungsbewegung vermessen werden kann. Der Innendruck überschreitet beispielsweise diesen Grenzwert, wenn ein Einatmen des Patienten vorliegt, wobei der Innendruck zwischen den einzelnen Atmungsbewegungen des Patienten wieder unterhalb des Grenzwertes abfällt. Bei Überschreiten des Grenzwertes durch den Innendruck des Druckkissens kann somit auf das Vorliegen einer Atmungsbewegung des Patienten geschlossen werden. Eine atmungsabhängige Bildaufnahme kann auf diese Weise durch das Messsignal getriggert werden. Selbstverständlich ist es möglich, dass auch andere Verfahren angewendet werden, um aus der Druckverlaufsmessung das Messsignal zu erzeugen.

Beispielsweise ist eine komplexere Analyse der Druckverlaufskurve des Innendrucks möglich. Hierbei ist es auch denkbar, falls deutlich genug in der Druckverlaufskurve enthalten, zusätzlich eine Information zum Herzschlag des Patienten abzuleiten.

Für ein erfindungsgemäßes Verfahren zur Aufnahme von medizinischen Bilddaten eines Patienten ist erfindungsgemäß vorgesehen, dass ein Messsignal durch ein erfindungsgemäßes Verfahren zur Messung eines wenigstens eine Atmungsbewegung eines auf einer Patientenlagerungseinrichtung gelagerten Patienten beschreibenden Messignals bestimmt wird und dass wenigstens eine Auslösung einer Bilddatenaufnahme in Abhängigkeit des Messsignals des Patienten erfolgt und/oder wenigstens eine Auswertung der Bilddaten unter Verwendung des Messsignals erfolgt. Wie vorangehend beschrieben wurde, kann das Messsignal zur Auslösung bzw. Triggerung einer oder mehrerer Bilddatenaufnahmen verwendet werden. Es ist auch möglich, dass das Messsignal aufgezeichnet wird, wobei anschließend eine Auswertung der Bilddaten in Abhängigkeit des Messsignals erfolgt. Dabei kann beispielsweise durch die Verwendung von Zeitstempeln in den Bilddaten und bei dem Messsignal eine Zuordnung der aufgenommenen Bilddaten zu der durch das Messsignal beschriebenen Atmungsbewegung des Patienten erfolgen.

Für eine erfindungsgemäße Messeinrichtung ist vorgesehen, dass sie eine Recheneinrichtung und eine Druckkisseneinrichtung umfasst, wobei die Druckkisseneinrichtung ein komprimierbares Druckkissen, in dessen Inneren ein Innendruck vorhanden ist oder erzeugbar ist, sowie einen mit dem Inneren des Druckkissens kommunizierenden Drucksensor umfasst, wobei die Recheneinrichtung zur Durchführung eines erfindungsgemäßen Verfahrens zur Messung einer wenigstens eine Atmungsbewegung eines auf einer Patientenlagerungseinrichtung gelagerten Patienten beschreibenden Messsignals ausgebildet ist. Durch die Recheneinrichtung kann eine gegebenenfalls vorhandene Pumpe angesteuert werden, wobei insbesondere über die Pumpe verschieden hohe Innendrücke des Druckkissens erzeugbar sind. Weiterhin ist es möglich, dass die Recheneinrichtung eine gegebenenfalls vorhandene Patienteninformation auswertet und die Pumpe in Abhängigkeit des Auswerteergebnisses zur Erzeugung eines Innendrucks ansteuert. Es ist auch möglich, dass über die Recheneinrichtung eine Kompensation des Einflusses eines einen Herzschlag des Patienten beschreibenden Pulssignals bei der Druckverlaufsmessung ausgebildet ist und/oder dass die Recheneinrichtung zur Auswertung der Druckverlaufsmessung und zur Bestimmung des Messsignals ausgebildet ist.

Für eine erfindungsgemäße medizinische Bildgebungseinrichtung ist vorgesehen, dass sie eine Patientenlagerungseinrichtung sowie eine erfindungsgemäße Messeinrichtung umfasst, wobei die Recheneinrichtung zusätzlich zur Durchführung eines erfindungsgemäßen Verfahrens zur Aufnahme von medizinischen Bilddaten eines Patienten ausgebildet ist. Die Recheneinrichtung kann dabei z. B. eine Steuerungseinrichtung der Bildgebungseinrichtung sein. Die Bildgebungseinrichtung kann beispielsweise ein Computertomographiegerät oder ein Magnetresonanztomographiegerät sein.

Weitere Vorteile und Einzelheiten der Erfindung ergeben sich aus den im Folgenden beschriebenen Ausführungsbeispielen sowie anhand der Zeichnungen. Dabei zeigen:
- Fig. 1: eine schematische Darstellung eines Ausführungsbeispiels einer erfindungsgemäßen Messeinrichtung,
- Fig. 2: eine schematische Darstellung eines weiteren Ausführungsbeispiels einer erfindungsgemäßen Messeinrichtung,
- Fig. 3: eine schematische Darstellung einer erfindungsgemäßen medizinischen Bildgebungseinrichtung,
- Fig. 4: eine schematische Darstellung einer Druckverlaufsmessung eines Innendrucks eines Druckkissens,
- Fig. 5: eine schematische Darstellung der Kompensation eines Pulssignales, sowie
- Fig. 6: ein Ablaufdiagramm eines erfindungsgemäßen Verfahrens zur Messung einer Atmungsbewegung.

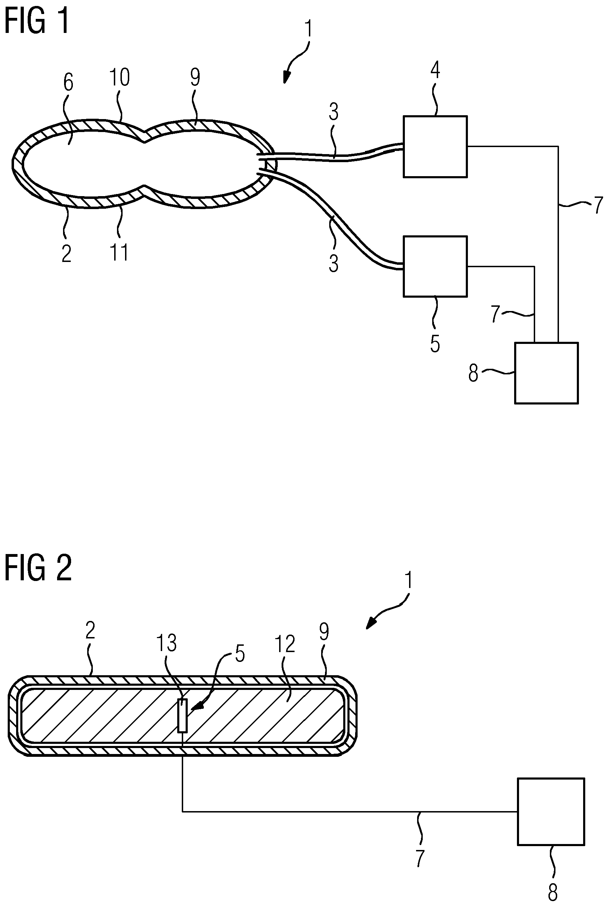

In Fig. 1 ist eine schematische Darstellung einer erfindungsgemäßen Messeinrichtung 1 abgebildet. Die Messeinrichtung umfasst ein komprimierbares Druckkissen 2, welches über jeweils einen Schlauch 3 mit einer Pumpe 4 sowie einem Drucksensor 5 verbunden ist. Der Drucksensor 5 kommuniziert über den Schlauch 3 mit einem Inneren 6 des Druckkissens 2. Im Inneren des Druckkissens 2 herrscht ein Innendruck, welcher beispielsweise über die Pumpe 4 als Luftdruck erzeugt werden kann. Der Innendruck des Luftkissens 2 kann beispielsweise zwischen 100 mbar und 400 mbar betragen. Der im Inneren 6 des Druckkissens 2 herrschende Innendruck kann über den Drucksensor 5 gemessen werden, insbesondere kann ein zeitlicher Verlauf des Innendrucks innerhalb eines Messzeitraumes durch den Drucksensor 5 bestimmt werden. Die Pumpe 4 sowie der Drucksensor 5 sind über elektrische Verbindemittel 7 mit einer Recheneinrichtung 8 der Messeinrichtung 1 verbunden. Die Recheneinrichtung 8 steuert die Pumpe 4 zur Erzeugung eines Innendrucks im Inneren 6 des Druckkissens 2 an. Weiterhin ist die Recheneinrichtung 8 dazu ausgebildet, aus der von dem Drucksensor 5 erzeugten Druckverlaufsmessung des im Inneren 6 des Druckkissens 2 herrschenden Innendrucks ein Messsignal zu bestimmen, welches bei Anordnung des Druckkissens 2 zwischen einem Patienten und einer Patientenlagerungseinrichtung eine Atmungsbewegung des Patienten beschreibt.

Das Druckkissen 2 ist aufblasbar und umfasst eine Hülle 9 aus Kunststoff, wobei der Kunststoff derart gewählt ist, dass das Druckkissen eine Messung in einer Röntgenbildgebungsmodalität und/oder in einer Magnetresonanzbildgebungsmodalität nicht beeinflusst. Die Schläuche 3, welche das Druckkissen 2 mit der Pumpe 4 sowie dem Drucksensor 5 verbinden, können ebenfalls aus einem entsprechenden Kunststoff hergestellt sein. Das Druckkissen 2 kann bevorzugt so dimensioniert sein, dass es in einem zumindest teilweise aufgeblasenen Zustand eine Dicke zwischen 0,1 cm und 10 cm aufweist. Eine zum Patienten weisende Oberfläche 10 des Druckkissens 2 sowie die Kontaktfläche 11 zur Patientenlagerungseinrichtung in einem angeordneten Zustand des Druckkissens können jeweils einen Flächeninhalt zwischen 50 cm² und 200 cm² aufweisen. Das Druckkissen 2 kann beispielsweise eine zylinder- oder zylinderscheibenförmige Form oder eine Quaderform aufweisen.

In Fig. 2 ist ein alternatives Ausführungsbeispiel einer erfindungsgemäßen Messeinrichtung 1 gezeigt. Das Druckkissen 2 umfasst eine Hülle 9 aus Kunststoff, wobei sich im Inneren 6 des Druckkissens ein Schaumstoffkörper 12 befindet. Der Schaumstoffkörper 12 kann dabei im Inneren 6 des Druckkissens 2 in einem gewissen Maße komprimiert sein, so dass durch den Schaumstoffkörper 12 ein Innendruck des Druckkissens 2 erzeugt wird. Der Schaumstoffkörper 12 ist dabei nicht vollständig komprimiert, so dass eine Komprimierbarkeit des Druckkissens 2 beibehalten wird. Das Druckkissen 2 umfasst weiterhin einen Drucksensor 5, welcher einen druckempfindlichen Widerstand 13 umfasst. Der druckempfindliche Widerstand 13 ist über das elektrische Verbindungsmittel 7 mit der Recheneinrichtung 8 verbunden und dient der Messung des Innendrucks im Druckkissen 2. Es ist möglich, dass der Drucksensor 5 mehr als einen druckempfindlichen Widerstand 13 aufweist, welche beispielsweise beabstandet voneinander im Inneren 6 des Druckkissens 2 angeordnet sein können.

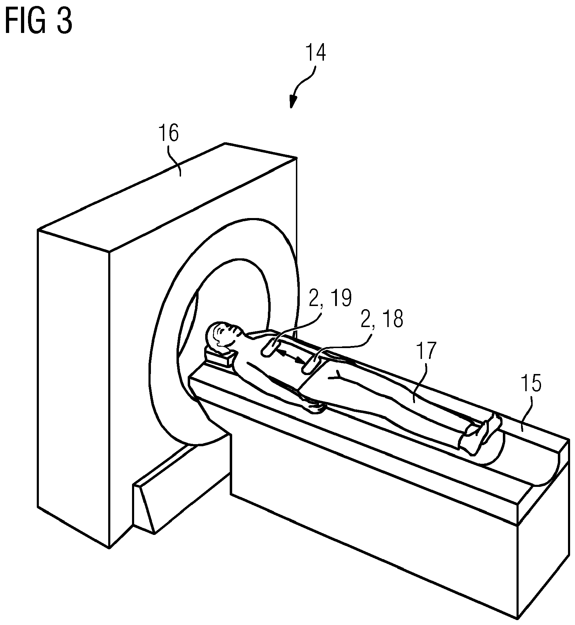

In Fig. 3 ist eine erfindungsgemäße medizinische Bildgebungseinrichtung 14 dargestellt. Die medizinische Bildgebungseinrichtung 14 umfasst dabei eine als Patientenliege ausgebildete Patientenlagerungseinrichtung 15 sowie eine Gantry 16, in welche ein Patient 17 zur Aufnahme von Bilddaten mittels der medizinischen Bildgebungseinrichtung 14 eingebracht werden kann. Weiterhin umfasst die medizinische Bildgebungseinrichtung 14 eine erfindungsgemäße Messeinrichtung 1, von der in Fig. 3 der Übersichtlichkeit halber nur das Druckkissen 2 dargestellt ist. Das Druckkissen 2 ist in einer Position 18 unterhalb eines Rückens des Patienten 17 gegenüberliegend des Bauchs des Patienten 17 angeordnet. Alternativ ist auch eine Anordnung des Druckkissens 2 in einer Position 19 unterhalb des Rückens des Patienten 17 gegenüberliegend einer Brust des Patienten 17 möglich. Die Positionierung des Druckkissens 2 kann abhängig davon erfolgen, ob der Patient 17 eher im Rückbereich oder eher im Bauchbereich eine stärkere Atmungsbewegung zeigt.

Das Druckkissen 2 kann als separater Gegenstand ausgeführt sein, so dass es beliebig zwischen dem Patienten 17 und der Patientenlagerungseinrichtung 15 positioniert werden kann. Es ist auch möglich, dass die Patientenlagerungseinrichtung 15 ein oder mehrere Druckkissen 2 aufweist, welche beispielsweise an den Positionen 18, 19 in die Patientenlagerungseinrichtung 15 integriert sind.

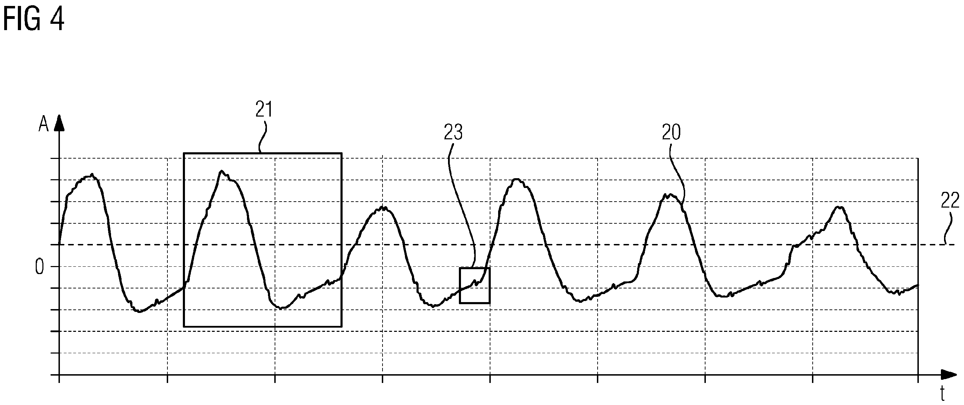

Aufgrund des Körpergewichtes des Patienten 17 und der stationären Lagerung des Druckkissens 2 auf der Patientenlagerungseinrichtung 15 wird das Druckkissen 2 teilweise komprimiert. Eine zusätzliche Kompression des Druckkissen 2 tritt bei einer Atmungsbewegung des Patienten 17 auf. Diese Kompression aufgrund der Atmungsbewegung hat eine Veränderung des Innendrucks des Druckkissens 2 zur Folge, welche über den Drucksensor 5 gemessen werden kann. Aus einer zeitlichen Druckverlaufsmessung kann durch die Recheneinrichtung 8 wenigstens eine Atmungsbewegung des Patienten 17 bestimmt werden, wodurch auf die jeweiligen Atemvorgänge des Patienten 17 zurückgeschlossen werden kann, wie nachfolgend in Bezug zu Fig. 4 beschrieben wird.

In Fig. 4 ist eine schematische Druckverlaufsmessung des Innendrucks des Druckkissens 2 dargestellt, wobei auf der Abszisse die Zeit und auf der Ordinate der zeitabhängige Druckverlauf des Innendrucks des Druckkissens 2 in Kurve 20 aufgetragen ist. Die Darstellung erfolgt dabei in willkürlichen Einheiten. Der zu einer einzelnen Atmungsbewegung des Patienten 17 korrespondierende Verlauf des Innendrucks ist durch den Kasten 21 markiert. Eine Bestimmung der Atmungsbewegung des Patienten 17 kann beispielsweise durch eine Grenzwerterkennung erfolgen. Ein Beispiel für einen Grenzwert wird durch die Linie 22 dargestellt. Wenn der im Inneren des Druckkissens gemessene Innendruck den Grenzwert übersteigt, liegt eine Atmungsbewegung des Patienten 17 vor. Mit Hilfe der Grenzwerterkennung kann somit aus der in Kurve 20 gezeigten Druckverlaufsmessung ein Messsignal bestimmt werden, welches angibt, wann eine Atmungsbewegung des Patienten 17 vorliegt.

Da auch der Herzschlag aufgrund der durch den systolischen Blutausstoß erzeugten Pulswelle eine Druckänderung des Druckkissens 2 hervorrufen kann, kann die Druckverlaufsmessung, wie in Kurve 20 dargestellt ist, durch von der Pulswelle erzeugte Störungen überlagert sein. Eine derartige Störung ist beispielsweise im Kasten 23 zu sehen.

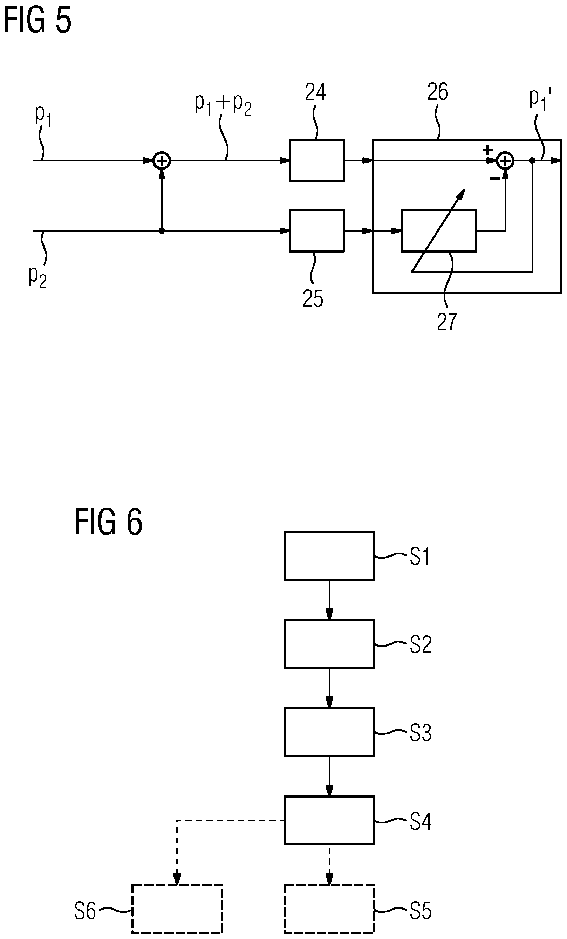

Um insbesondere im Fall einer nur geringen Atmungsbewegung des Patienten 17 eine Kompensation der durch die Pulswelle erzeugten Störung vorzunehmen, kann, wie in Fig. 5 dargestellt ist, eine Kompensation des Pulssignales mittels Echo-Cancellation erfolgen.

In Fig. 5 ist ein schematisches Blockdiagramm der Kompensation eines Pulssignals mittels Echo-Cancellation dargestellt. Dabei beschreibt p₁ den zeitlichen Druckverlauf, welcher sich aufgrund der Atmungsbewegung des Patienten 17 ergibt. Der zeitliche Druckverlauf, welcher sich aufgrund des Herzschlags des Patienten 17 ergibt, und das Signal p₁ als Störung überlagert, ist als p₂ bezeichnet. Eine Kompensation des Pulssignals p₂ kann durch eine separate Messung des Pulssignales, beispielsweise mittels eines Elektrokardiogramm-Signals und/oder einer pneumatischen Messung der Pulswelle erfolgen. Das durch das Pulssignal überlagerte Signal p₁ + p₂ wird durch einen Analog-Digital-Wandler 24 gewandelt. Entsprechend wird das separat gemessene Pulssignal p₂ durch einen Analog-Digital-Wandler 25 gewandelt. In Block 26 erfolgt eine Kompensation des Pulssignales p₂ mittels Echo-Cancellation unter Verwendung eines adaptiven Filters 27. Durch Ansteuerung des adaptiven Filters 27 mit dem digitalisierten Pulssignal p₂ sowie durch Rückkopplung des Ausgangssignals p₁' wird ein Ausgangssignal p₁' erhalten, welches im Wesentlichen vom Einfluss des Pulssignales p₂ befreit worden ist. Die Analog-Digital-Wandler 24, 25 sowie der adaptive Filter 27 können beispielswiese Bestandteile der Recheneinrichtung 8 der Messeinrichtung 1 sein. Auch die Funktion des Blocks 26 kann in der Recheneinrichtung 8 implementiert sein.

In Fig. 6 ist ein schematisches Flussdiagramm eines erfindungsgemäßen Verfahrens zur Messung eines wenigstens eine Atembewegung eines auf einer Patientenlagerungseinrichtung 15 gelagerten Patienten 17 beschreibenden Messsignals dargestellt. Dabei erfolgt in Schritt S1 eine Positionierung des Druckkissens 2 zwischen dem Patienten 17 und der Patientenlagerungseinrichtung 15. Diese Positionierung kann insbesondere unter einen Rücken des Patienten 17 gegenüberliegend der Brust des Patienten oder des Bauches des Patienten erfolgen, wie vorangehend in Bezug zu Fig. 3 beschrieben wurde.

Anschließend erfolgt in Schritt S2 ein Aufbau des Innendrucks im Inneren 6 des Druckkissens 2 durch die Pumpe 4 in dem Fall, dass ein aufblasbares Druckkissen verwendet wird. Der Innendruck des Druckkissens 2 kann beispielsweise zwischen 100 mbar und 400 mbar betragen, wobei der Wert des Innendrucks in diesem Intervall in Abhängigkeit einer Patienteninformation, welche wenigstens eine Eigenschaft des Patienten 17 beschreibt, gewählt wird. Die Patienteninformation kann beispielsweise eine Größe des Patienten 17, ein Geschlecht des Patienten 17 und/oder ein Alter des Patienten 17 beschreiben, wobei der Innendruck im Druckkissen 2 in Abhängigkeit wenigstens einer dieser Eigenschaften gewählt wird.

In Schritt S3 erfolgt anschießend die Messung wenigstens einer Atmungsbewegung des Patienten durch eine Druckverlaufsmessung, welche durch den Drucksensor 5 durchgeführt wird. In Schritt S4 wird aus der in Schritt S3 gewonnenen Druckverlaufsmessung ein Messsignal erzeugt. Dabei ist es insbesondere möglich, dass die Bestimmung des Messsignals kontinuierlich während der Druckverlaufsmessung erzeugt wird, wodurch eine Erzeugung des Messsignals kontinuierlich und ohne nennenswerten Zeitversatz während der Druckverlaufsmessung ermöglicht wird. Es ist weiterhin möglich, dass bei der Bestimmung des Messsignals, wie vorangehend im Bezug zu den Figuren 4 und 5 beschrieben wurde, eine Kompensation eines Pulssignals vorgenommen wird.

Das erfindungsgemäße Verfahren zur Messung der Atmungsbewegung, welches die Schritte S1 - S4 umfasst, kann Bestandteil eines erfindungsgemäßen Verfahrens zur Aufnahme von medizinischen Bilddaten eines Patienten sein, wobei das in Schritt S4 bestimmte Messsignal in einem Schritt S5 des Verfahrens zur Aufnahme von medizinischen Bilddaten zur Triggerung einer Bildaufnahme einer medizinischen Bildgebungseinrichtung verwendet wird. Zusätzlich oder alternativ dazu kann in einem Schritt S6 des erfindungsgemäßen Verfahrens zur Aufnahme von medizinischen Bilddaten eine Speicherung der Druckverlaufsmessung und/oder des in Schritt S4 erzeugten Messsignals erfolgen, wodurch eine Auswertung der Bilddaten unter Verwendung des Messsignals ermöglicht wird. Dadurch wird beispielsweise eine nachträgliche Korrektur der erhaltenen Bilddaten um die aufgrund der Atmungsbewegung eintretenden Bewegungsartefakte ermöglicht.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Messung eines wenigstens eine Atmungsbewegung eines auf einer Patientenlagerungseinrichtung (15) gelagerten Patienten (17) beschreibenden Messsignals, **dadurch gekennzeichnet, dass** auf der Patientenlagerungseinrichtung (15) zumindest bereichsweise unterhalb des Patienten (17) ein komprimierbares Druckkissen (2) einer Druckkisseneinrichtung angeordnet wird, wobei in einem Inneren (6) des Druckkissens (2) ein Innendruck vorhanden ist oder erzeugt wird und das Messsignal aus einer Druckverlaufsmessung des Innendrucks eines mit dem Inneren (6) des Druckkissens (2) kommunizierenden Drucksensors (5) der Druckkisseneinrichtung bestimmt wird.

2. Verfahren nach Anspruch 1, **dadurch gekennzeichnet, dass** das Druckkissen (2) zumindest bereichsweise unterhalb eines Rückens des Patienten (17), insbesondere gegenüber einer Brust oder eines Bauchs des Patienten (17), angeordnet wird.

3. Verfahren nach Anspruch 1 oder 2, **dadurch gekennzeichnet, dass** der Innendruck durch einen Schaumstoffkörper (12) des Druckkissens (2) erzeugt wird.

4. Verfahren nach Anspruch 3, **dadurch gekennzeichnet, dass** als Drucksensor (5) wenigstens ein druckempfindlicher Widerstand (13) verwendet wird.

5. Verfahren nach Anspruch 1 oder 2, **dadurch gekennzeichnet, dass** ein aufblasbares Druckkissen (2) verwendet wird, wobei der Innendruck durch eine Pumpe (4) der Druckkisseneinrichtung erzeugt wird.

6. Verfahren nach Anspruch 5, **dadurch gekennzeichnet, dass** eine Höhe des Innendrucks in Abhängigkeit einer wenigstens eine Eigenschaft des Patienten (17) beschreibenden Patienteninformation bestimmt wird.

7. Verfahren nach Anspruch 5 oder 6, **dadurch gekennzeichnet, dass** ein Innendruck zwischen 100 mbar und 400 mbar verwendet wird.

8. Verfahren nach einem der Ansprüche 5 bis 7, **dadurch gekennzeichnet, dass** ein aufblasbares Druckkissen (2) verwendet wird, dessen Kontaktfläche (11) zu der Patientenlagerungseinrichtung (15) und/oder dessen zum Patienten (17) weisende Oberfläche (10) jeweils in zumindest teilweise aufgeblasenem Zustand zwischen 50 cm² und 200 cm² beträgt und/oder dessen Dicke in zumindest teilweise aufgeblasenem Zustand zwischen 0,1 cm und 10 cm beträgt.

9. Verfahren nach einem der vorangehenden Ansprüche, **dadurch gekennzeichnet, dass** ein Druckkissen (2) aus einem röntgentransparenten Material und/oder einem amagnetischen Material, insbesondere aus Kunststoff, verwendet wird.

10. Verfahren nach einem der vorangehenden Ansprüche, **dadurch gekennzeichnet, dass** ein Druckkissen (2) verwendet wird, welches in die Patientenlagerungseinrichtung (15) integriert ist.

11. Verfahren nach einem der vorangehenden Ansprüche, **dadurch gekennzeichnet, dass** die Druckverlaufsmessung um den Einfluss eines einen Herzschlag des Patienten (17) beschreibenden Pulssignals, insbesondere eines Elektrokardiogramm-Signals und/oder eines pneumatisch gemessenen Pulssignals, kompensiert wird.

12. Verfahren nach einem der vorangehenden Ansprüche, **dadurch gekennzeichnet, dass** das Messsignal durch eine Grenzwerterkennung des von dem Drucksensor (5) gemessenen Innendrucks des Druckkissens (2) erfolgt.

13. Verfahren zur Aufnahme von medizinischen Bilddaten eines Patienten (17), wobei ein Messsignal durch ein Verfahren nach einem der vorangegangen Ansprüche bestimmt wird und wobei wenigstens eine Auslösung einer Bilddatenaufnahme in Abhängigkeit des Messsignals des Patienten (17) erfolgt und/oder wenigstens eine Auswertung der Bilddaten unter Verwendung des Messsignals erfolgt.

14. Messeinrichtung, umfassend eine Recheneinrichtung (8) und eine Druckkisseneinrichtung, wobei die Druckkisseneinrichtung ein komprimierbares Druckkissen (2), in dessen Inneren (6) ein Innendruck vorhanden ist oder erzeugbar ist, sowie einen mit dem Inneren (6) des Druckkissens (2) kommunizierenden Drucksensor (5) umfasst, wobei die Recheneinrichtung (8) zur Durchführung eines Verfahrens nach einem der Ansprüche 1 bis 12 ausgebildet ist.

15. Medizinische Bildgebungseinrichtung, umfassend eine Patientenlagerungseinrichtung (15) und eine Messeinrichtung (1) nach Anspruch 14, wobei die Recheneinrichtung (8) zusätzlich zur Durchführung eines Verfahrens nach Anspruch 13 ausgebildet ist.
